# EUROPÄISCHE PATENTSCHRIFT

(11) **EP 0 763 028 B1**
(45) Veröffentlichungstag und Bekanntmachung des Hinweises auf die Patenterteilung: **16.09.1998**
(21) Anmeldenummer: 95920821.6
(22) Anmeldetag: 17.05.1995
(51) Int. Cl.: C07D 239/42, A01N 47/36, C07D 251/12, C07D 251/16, C07D 251/46, C07D 239/46, C07D 239/52

(54) **FORMYLAMINOPHENYLSULFONYLHARNSTOFFE, VERFAHREN ZUR HERSTELLUNG UND VERWENDUNG ALS HERBIZIDE UND PFLANZENWACHSTUMSREGULATOREN**
FORMYLAMINOPHENYLSULPHONYL UREAS, METHODS OF PREPARING THEM AND THEIR USE AS HERBICIDES AND PLANT-GROWTH REGULATORS
FORMYLAMINOPHENYLSULFONYLUREES, LEUR PROCEDE DE FABRICATION ET LEUR UTILISATION EN TANT QU'HERBICIDES ET REGULATEURS DE CROISSANCE DES VEGETAUX

(30) Priorität: 01.06.1994 DE 4419259; 20.03.1995 DE 19510078
(43) Veröffentlichungstag der Anmeldung: 19.03.1997
(73) Patentinhaber: Hoechst Schering AgrEvo GmbH, 13509 Berlin (DE)
(72) Erfinder: SCHNABEL, Gerhard, D-63868 Gro wallstadt (DE); WILLMS, Lothar, D-65719 Hofheim (DE); BAUER, Klaus, D-63456 Hanau (DE); BIERINGER, Hermann, D-65817 Eppstein (DE); ROSINGER, Christopher, D-65719 Hofheim (DE)
(86) Internationale Anmeldenummer: EP9501867
(87) Internationale Veröffentlichungsnummer: WO9532950

(56) Entgegenhaltungen:
- EP-A- 0 116 518
- DE-A- 4 236 902
- US-A- 4 892 946

## Beschreibung

Die Erfindung betrifft das technische Gebiet der Herbizide und Pflanzenwachstumsregulatoren, insbesondere der Herbizide zur selektiven Bekämpfung von Unkräutern und Ungräsern in Nutzpflanzenkulturen.

Es ist bekannt, daß heterocyclisch substituierte Phenylsulfonylharnstoffe, die am Phenylring eine Amino- bzw. eine funktionalisierte Aminogruppe tragen, herbizide und pflanzenwachstumsregulierende Eigenschaften besitzen; siehe EP-A-1515, US-A-4892946, US-A-4981509, DE-A- 4322067, EP-A-116518 (= US-A-4664695, US-A-4632695), DE-A-4236902 (WO 94/10154).

Überraschenderweise wurde nun gefunden, daß bestimmte substituierte N-(Aminophenylsulfonyl)-N'-(pyrimidinyl- oder -triazinyl)-harnstoffe als Herbizide oder Pflanzenwachstumsregulatoren besonders gut geeignet sind.

Gegenstand der vorliegenden Erfindung sind Verbindungen der Formel (I) 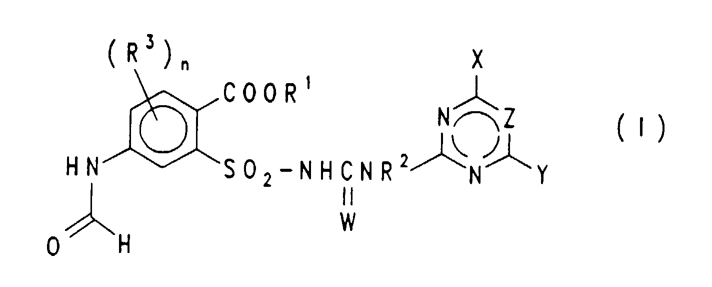 worin
- R¹: H, einen substituierten oder unsubstituierten Kohlenwasserstoffrest oder einen unsubstituierten oder substituierten heterocyclischen Rest,
- R²: H, (C₁-C₆)-Alkyl oder (C₁-C₆)-Alkoxy,
- R³: Halogen, (C₁-C₆)-Alkyl,(C₁-C₆)-Alkoxy, (C₁-C₆)-Haloalkyl, (C₁-C₆)-Haloalkoxy, NO₂, CN, NH₂, (C₁-C₄)-Mono- oder Dialkylamino, und zwar jeweils unabhängig von anderen Resten R³, wenn n 2 oder 3 ist,
- n: 0, 1, 2 oder 3,
- W: ein Sauerstoffatom oder ein Schwefelatom,
- X, Y: unabhängig voneinander Halogen, (C₁-C₆)-Alkyl, (C₁-C₆)-Alkoxy, (C₁-C₆)-Alkylthio, wobei jeder der drei letztgenannten Reste unsubstituiert oder durch einen oder mehrere Reste aus der Gruppe Halogen, (C₁-C₄)-Alkoxy und (C₁-C₄)-Alkylthio substituiert ist, oder (C₃-C₆)-Cycloalkyl, (C₂-C₆)-Alkenyl, (C₂-C₆)-Alkinyl, (C₂-C₆)-Alkenyloxy, (C₂-C₆)-Alkinyloxy, Mono-oder Di-[(C₁-C₄)-alkyl]-amino und
- Z: CH oder N bedeuten.

In Formel (I) und allen nachfolgenden Formeln können die Reste Alkyl, Alkoxy, Haloalkyl, Haloalkoxy, Alkylamino und Alkylthio sowie die entsprechenden ungesättigten und/oder substituierten Reste im Kohlenstoffgerüst jeweils geradkettig oder verzweigt sein. Wenn nicht speziell angegeben, sind bei diesen Resten die niederen Kohlenstoffgerüste, z.B. mit 1 bis 4 C-Atomen bzw. bei ungesättigten Gruppen mit 2 bis 4 C-Atomen, bevorzugt. Alkylreste, auch in den zusammengesetzten Bedeutungen wie Alkoxy, Haloalkyl usw., bedeuten z.B. Methyl, Ethyl, n- oder i-Propyl, n-, i-, t- oder 2-Butyl, Pentyle, Hexyle, wie n-Hexyl, i-Hexyl und 1,3-Dimethylbutyl, Heptyle, wie n-Heptyl, 1-Methylhexyl und 1,4-Dimethylpentyl; Alkenyl- und Alkinylreste haben die Bedeutung der den Alkylresten entsprechenden möglichen ungesättigten Reste; Alkenyl bedeutet z.B. Allyl, 1-Methylprop-2-en-1-yl, 2-Methyl-prop-2-en-1-yl, But-2-en-1-yl, But-3-en-1-yl, 1-Methyl-but-3-en-1-yl und 1-Methyl-but-2-en-1-yl; Alkinyl bedeutet z.B. Propargyl, But-2-in-1-yl, But-3-in-1-yl, 1-Methyl-but-3-in-1-yl.

Halogen bedeutet beispielsweise Fluor, Chlor, Brom oder lod. Haloalkyl, -alkenyl und -alkinyl bedeuten durch Halogen, vorzugsweise durch Fluor, Chlor und/oder Brom, insbesondere durch Fluor oder Chlor, teilweise oder vollständig substituiertes Alkyl, Alkenyl bzw. Alkinyl, z.B. CF₃, CHF₂, CH₂F, CF₃CF₂, CH₂FCHCl, CCl₃, CHCl₂, CH₂CH₂Cl; Haloalkoxy ist z.B. OCF₃, OCHF₂, OCH₂F, CF₃CF₂O, OCH₂CF₃ und OCH₂CH₂Cl; entsprechendes gilt für Haloalkenyl und andere durch Halogen substituierte Reste.

Ein Kohlenwasserstoffrest ist ein geradkettiger, verzweigter oder cyclischer und gesättigter oder ungesättigter aliphatischer oder aromatischer Kohlenwasserstoffrest, z.B. Alkyl, Alkenyl, Alkinyl, Cycloalkyl, Cycloalkenyl oder Aryl; Aryl bedeutet dabei ein mono-, bi- oder polycyclisches aromatisches System, beispielsweise Phenyl, Naphthyl, Tetrahydronaphthyl, Indenyl, Indanyl, Pentalenyl, Fluorenyl und ähnliches, vorzugsweise Phenyl; vorzugsweise bedeutet ein Kohlenwasserstoffrest Alkyl, Alkenyl oder Alkinyl mit bis zu 12 C-Atomen oder Cycloalkyl mit 5 oder 6 Ringatomen oder Phenyl;

Ein heterocyclischer Rest oder Ring kann gesättigt, ungesättigt oder heteroaromatisch sein; er enthält ein oder mehrere Heteroringatome, vorzugsweise aus der Gruppe N, O und S; vorzugsweise ist er 5- oder 6-gliedrig und enthält 1, 2 oder 3 Heteroringatome. Der heterocyclische Rest kann z.B. ein heteroaromatischer Rest oder Ring (Heteroaryl) sein, wie z.B. ein mono-, bi- oder polycyclisches aromatisches System, in dem mindestens 1 Ring ein oder mehrere Heteroatome enthält, beispielsweise Pyridyl, Pyrimidinyl, Pyridazinyl, Pyrazinyl, Thienyl, Thiazolyl, Oxazolyl, Furyl, Pyrrolyl, Pyrazolyl und Imidazolyl, oder ist ein partiell hydrierter Rest wie Oxiranyl, Pyrrolidyl, Piperidyl, Piperazinyl, Dioxolanyl, Morpholinyl, Tetrahydrofuryl. Als Substituenten für einen substituierten heterocyclischen Rest kommen die weiter unten genannten Substituenten in Frage, zusätzlich auch Oxo. Die Oxogruppe kann auch an den Heteroringatomen, die in verschiedenen Oxidationsstufen existieren können, z.B. bei N und S, auftreten.

Substituierte Reste, wie substituierte Kohlenwasserstoffreste, z.B. substituiertes Alkyl, Alkenyl, Alkinyl, Aryl, Phenyl und Benzyl, oder substituiertes Heteroaryl, bedeuten beispielsweise einen vom unsubstituierten Grundkörper abgeleiteten substituierten Rest, wobei die Substituenten beispielsweise einen oder mehrere, vorzugsweise 1, 2 oder 3 Reste aus der Gruppe Halogen, Alkoxy, Haloalkoxy, Alkylthio, Hydroxy, Amino, Nitro, Cyano, Azido, Alkoxycarbonyl, Alkylcarbonyl, Formyl, Carbamoyl, Mono- und Dialkylaminocarbonyl, substituiertes Amino wie Acylamino, Mono- und Dialkylamino, und Alkylsulfinyl, Haloalkylsulfinyl, Alkylsulfonyl, Haloalkylsulfonyl und, im Falle cyclischer Reste, auch Alkyl und Haloalkyl sowie den genannten gesättigten kohlenwasserstoffhaltigen Resten entsprechende ungesättigte aliphatische Reste, wie Alkenyl, Alkinyl, Alkenyloxy, Alkinyloxy etc. bedeuten. Bei Resten mit C-Atomen sind solche mit 1 bis 4 C-Atomen, insbesondere 1 oder 2 C-Atomen, bevorzugt. Bevorzugt sind in der Regel Substituenten aus der Gruppe Halogen, z.B. Fluor und Chlor, (C₁C₄)-Alkyl, vorzugsweise Methyl oder Ethyl, (C₁C₄)-Haloalkyl, vorzugsweise Trifluormethyl, (C₁-C₄)-Alkoxy, vorzugsweise Methoxy oder Ethoxy, (C₁-C₄)-Haloalkoxy, Nitro und Cyano. Besonders bevorzugt sind dabei die Substituenten Methyl, Methoxy und Chlor.

Gegebenenfalls substituiertes Phenyl ist vorzugsweise Phenyl, das unsubstituiert oder ein- oder mehrfach, vorzugsweise bis zu dreifach durch gleiche oder verschiedene Reste aus der Gruppe Halogen, (C₁-C₄)-Alkyl, (C₁-C₄)-Alkoxy, (C₁-C₄)-Halogenalkyl, (C₁-C₄)-Halogenalkoxy und Nitro substituiert ist, z.B. o-, m- und p-Tolyl, Dimethylphenyle, 2-, 3- und 4-Chlorphenyl, 2-, 3- und 4-Trifluor-und -Trichlorphenyl, 2,4-, 3,5-, 2,5- und 2,3-Dichlorphenyl, o-, m- und p-Methoxyphenyl.

Ein Acylrest bedeutet den Rest einer organischen Säure, z.B. den Rest einer Carbonsäure und Reste davon abgeleiteter Säuren wie der Thiocarbonsäure, gegebenenfalls N-substituierter Iminocarbonsäuren, oder der Rest von Kohlensäuremonoestern, gegebenenfalls N-substituierter Carbaminsäure, Sulfonsäuren, Sulfinsäuren, Phosphonsäuren, Phosphinsäuren. Acyl bedeutet beispielsweise Formyl, Alkylcarbonyl wie [(C₁-C₄)-Alkyl]-carbonyl, Phenylcarbonyl, wobei der Phenylring substituiert sein kann, z.B. wie oben für Phenyl gezeigt, oder Alkyloxycarbonyl, Phenyloxycarbonyl, Benzyloxycarbonyl, Alkylsulfonyl, Alkylsulfinyl, N-Alkyl-1-iminoalkyl und andere Reste von organischen Säuren.

Gegenstand der Erfindung sind auch alle Stereoisomeren, die von Formel (I) umfaßt sind, und deren Gemische. Solche Verbindungen der Formel (I) enthalten ein oder mehrere asymmetrische C-Atome oder auch Doppelbindungen, die in den allgemeinen Formeln (I) nicht gesondert angegeben sind. Die durch ihre spezifische Raumform definierten möglichen Stereoisomeren, wie Enantiomere, Diastereomere, Z- und E-lsomere sind alle von der Formel (I) umfaßt und können nach üblichen Methoden aus Gemischen der Stereoisomeren erhalten oder auch durch stereoselektive Reaktionen in Kombination mit dem Einsatz von stereochemisch reinen Ausgangsstoffen hergestellt werden.

Von besonderem Interesse sind erfindungsgemäße Verbindungen der Formel (I), worin
- R¹: Wasserstoff, einen aliphatischen oder cycloaliphatischen Kohlenwasserstoffrest mit bis zu 24 C-Atomen, der unsubstituiert oder substituiert ist, oder einen unsubstituierten oder substituierten gesättigten heterocyclischen Rest mit 3 bis 7 Ringatomen,
vorzugsweise H, (C₁-C₆)-Alkyl, (C₂-C₆)-Alkenyl, (C₂-C₆)-Alkinyl, (C₃-C₆)-Cycloalkyl, wobei die vier letztgenannten Reste unsubstituiert oder durch einen oder mehrere Reste aus der Gruppe Halogen, CN, (C₁-C₃)-Alkoxy, (C₁-C₃)-Haloalkoxy, (C₃-C₆)-Cycloalkyl, (C₁-C₃)-Alkylthio, (C₁-C₃)-Alkylsulfinyl, (C₁-C₃)-Alkylsulfonyl, unsubstituiertes Phenyl, substituiertes Phenyl, unsubstituierter heterocyclischer Rest und substituierter heterocyclischer Rest und im Falle cyclischer Reste auch (C₁-C₄)-Alkyl substituiert sind,
oder einen heterocyclischen Rest mit 3, 4, 5, 6 oder 7 Ringatomen und einem oder mehreren Atomen aus der Gruppe O, N und S als Heteroringatom, vorzugsweise ein gesättigter heterocyclischer Rest und vorzugsweise ein Sauerstoffatom als Heteroringatom, wobei der Rest unsubstituiert oder durch einen oder mehrere Reste aus der Gruppe Halogen, (C₁-C₃)-Alkyl und (C₁-C₃)-Haloalkyl substituiert ist,
- R²: H, (C₁-C₃)-Alkoxy, (C₁-C₃)-Alkyl,
- R³: Halogen, (C₁-C₃)-Alkyl, (C₁-C₃)-Alkoxy, (C₁-C₃)-Haloalkyl, (C₁-C₃)-Haloalkoxy, NO₂, CN, NH₂, NHCH₃, N(CH₃)₂,
- n: 0, 1, 2 oder 3, vorzugsweise 0, 1 oder 2, insbesondere 0 oder 1,
- W: O oder S, vorzugsweise O,
- X, Y: unabhängig voneinander Halogen, (C₁-C₄)-Alkyl, (C₁-C₄)-Alkoxy, (C₁-C₄)-Alkylthio, wobei jeder der drei letztgenannten Reste unsubstituiert oder durch einen oder mehrere Reste aus der Gruppe Halogen, (C₁-C₄)-Alkoxy und (C₁-C₄)-Alkylthio substituiert ist, oder (C₃-C₆)-Cycloalkyl, (C₂-C₄)-Alkenyl, (C₂-C₄)-Alkinyl, (C₂-C₄)-Alkenyloxy, (C₂-C₄)-Alkinyloxy, Mono-oder Di-[(C₁-C₂)-alkyl]-amino und
- Z: CH oder N bedeuten.

Bevorzugt sind erfindungsgemäße Verbindungen der Formel (I), worin R¹ (C₁-C₆)-Alkyl, (C₂-C₆)-Alkenyl, (C₂-C₆)-Alkinyl, (C₃-C₆)-Cycloalkyl, (C₄-C₈)-Cycloalkylalkyl, Phenyl-(C₁-C₆)-alkyl, wobei jeder der sechs letztgenannten Reste unsubstituiert oder durch einen oder mehrere Reste aus der Gruppe Halogen, wie F, Cl, Br und 1, CN, OCH₃, OC₂H₅, OCF₃, SO₂CH₃ und im Falle cyclischer Reste auch (C₁-C₃)-Alkyl substituiert ist, oder einen Rest der Formeln A₁ bis A₇, insbesondere A₁, A₂, A₃, 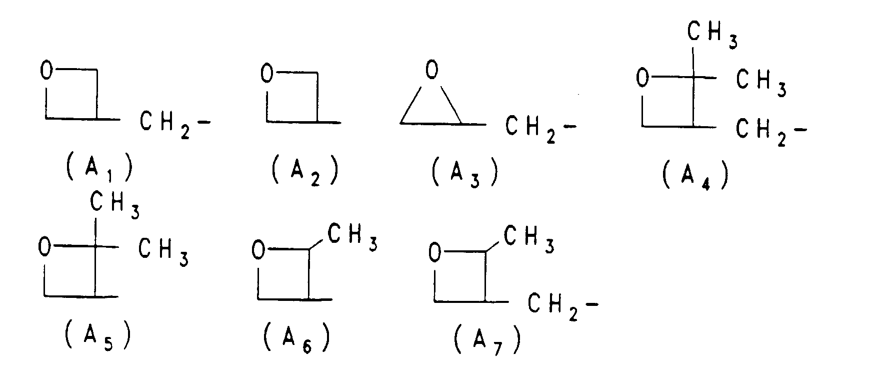
- R²: H oder CH₃,
- R³: Halogen, CH₃, C₂H₅, OCH₃, OC₂H₅, CF_{3'} CCl₃, OCF₃, OCHF₂ oder N(CH₃)₂,
- n: 0, 1 oder 2
bedeuten.

Weiterer Gegenstand der vorliegenden Erfindung sind Verfahren zur Herstellung der erfindungsgemäßen Verbindungen der Formel (I), dadurch gekennzeichnet, daß man
a) eine Verbindung der Formel (II) 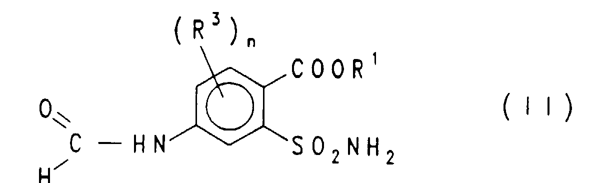 mit einem heterocyclischen Carbamat der Formel (III), 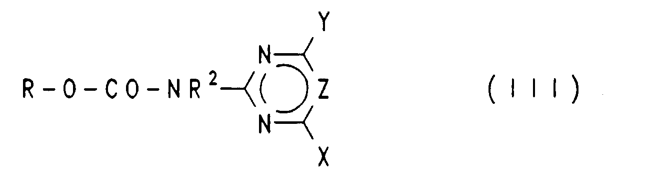 worin R unsubstituiertes oder substituiertes Phenyl bedeutet, umsetzt oder
b) ein Sulfochlorid der Formel (IV) mit einem 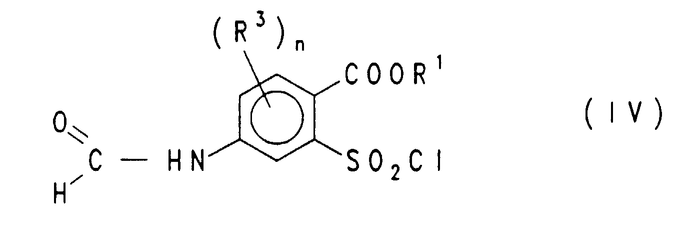 heterocyclischen Amin der Formel (V) 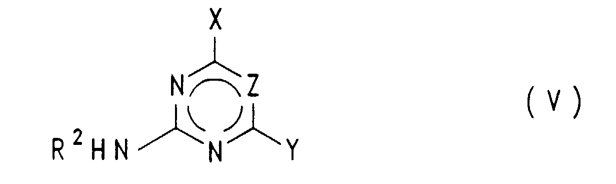 in Gegenwart eines Cyanats, z.B. eines Alkalimetallcyanats wie Natrium-oder Kaliumcyanat, umsetzt oder
c) einen Sulfonylharnstoff der Formel (Vl) 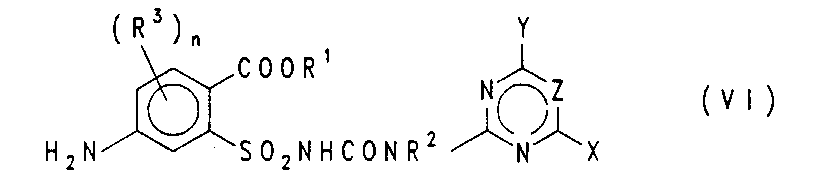 formyliert oder
d) ein Sulfonamid der Formel (II) mit einem (Thio)lsocyanat der Formel (VII) in Gegenwart einer geeigneten Base umsetzt, 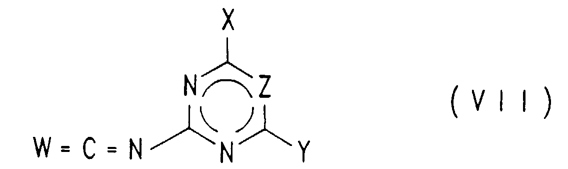 wobei in den obigen Formeln (II) bis (VII) die Reste R¹, R², R³, W, X, Y und Z sowie der Index n wie in Formel (I) definiert sind und in den Verfahrensvarianten a), b) und c) zunächst Verbindungen erhalten werden, in denen W ein Sauerstoffatom bedeutet.

Die Umsetzung der Verbindungen der Formeln (II) und (III) erfolgt vorzugsweise basenkatalysiert in inerten Solventien, wie z.B. Dichlormethan, Acetonitril, Dioxan, Dimethylformamid (DMF), Dimethylessigsäureamid oder Tetrahydrofuran (THF), bei Temperaturen von -10°C bis zum Siedepunkt des jeweiligen Lösungsmittels. Als Basen werden dabei beispielsweie organische Aminbasen wie 1,8-Diazabicyclo[5.4.0]undec-7-en (DBU), Triethylamin, oder auch Hydroxyde, wie z.B. Natrium- oder Kaliumyhydoxyd, oder Alkoholate, wie z.B. Natriummethylat, Kalium-tert.-butylat oder Natriumphenolat, oder Carbonate, wie z.B. Natrium- oder Kaliumcarbonat (vgl. z.B. EP-A-44807) verwendet.

Die Sulfonamide der Formel (II) sind neu und ebenfalls Gegenstand dieser Erfindung. Sie können analog bekannten Verfahren hergestellt werden, z. B. über die Formylierung entsprechender gegebenenfalls Schutzgruppen enthaltener Aminophenylsulfonamide.

Die für die Umsetzung benötigten Carbamate sind literaturbekannt oder lassen sich analog bekannten Verfahren herstellen (vgl. EP-A-70804; US-A-4,480,101 ; EP-A-562575; EP-A-562576).

Die Umsetzung der Sulfochloride (IV) mit den Aminoheterocyclen der Formel (V) und Cyanaten wie Natriumcyanat und Kaliumcyanat erfolgt z.B. in aprotischen Solventien, wie z.B. Acetonitril, gegebenenfalls in Gegenwart von Basen, z.B. 0,5 bis 2 Äquivalenten Base, oder in basischen aprotischen Solventien bei Temperaturen zwischen -10 und 100°C, vorzugsweise zwischen -10 und 60°C, insbesondere bei 15 bis 40°C. Als Base oder basische aprotische Solventien kommen z.B. Pyridin, Picolin oder Lutidin oder eine Mischung aus diesen in Betracht (vgl. US-A-5,157,119).

Die Umsetzung (Formylierung) von Sulfonylharnstoffen der Formel (Vl) zu den Verbindungen der Formel (I) erfolgt beispielsweise mit dem gemischten Anhydrid aus Ameisen- und Essigsäure bei Temperaturen von -10 bis 60°C, vorzugsweise bei 0 bis 40°C, entweder in Substanz oder in inerten Solventien wie z.B. Dichlormethan, Acetonitril oder Ethylacetat.

Die Reaktion der Sulfonamide der Formel (II) mit einem (Thio)lsocyanat der Formel (VII) erfolgt analog literaturbekannten Verfahren (z.B. EP-A-232067, EP-A-166516) bei -10 bis 150°C, vorzugsweise 20 bis 100°C, in einem inerten Lösungsmittel, wie z.B. Aceton oder Acetonitril, in Gegenwart einer geeigneten Base, wie z.B. Triethylamin oder Kaliumcarbonat.

Die erfindungsgemäßen Verbindungen der Formel (I) weisen eine ausgezeichnete herbizide Wirksamkeit gegen ein breites Spektrum wirtschaftlich wichtiger mono- und dikotyler Schadpflanzen auf. Auch schwer bekämpfbare perennierende Unkräuter, die aus Rhizomen, Wurzelstöcken oder anderen Dauerorganen austreiben, werden durch die Wirkstoffe gut erfaßt. Dabei ist es gleichgültig, ob die Substanzen im Vorsaat-, Vorauflauf- oder Nachauflaufverfahren ausgebracht werden. Im einzelnen seien beispielsweise einige Vertreter der mono- und dikotylen Unkrautflora genannt, die durch die erfindungsgemäßen Verbindungen kontrolliert werden können, ohne daß durch die Nennung eine Beschränkung auf bestimmte Arten erfolgen soll.

Auf der Seite der monokotylen Unkrautarten werden z.B. Avena, Lolium, Alopecurus, Phalaris, Echinochloa, Digitaria, Setaria sowie Cyperusarten aus der annuellen Gruppe und auf seiten der perennierenden Spezies Agropyron, Cynodon, Imperata sowie Sorghum und auch ausdauernde Cyperusarten gut erfaßt. Bei dikotylen Unkrautarten erstreckt sich das Wirkungsspektrum auf Arten wie z.B. Galium, Viola, Veronica, Lamium, Stellaria, Amaranthus, Sinapis, Ipomoea, Matricaria, Abutilon und Sida auf der annuellen Seite sowie Convolvulus, Cirsium, Rumex und Artemisia bei den perennierenden Unkräutern.

Unter den spezifischen Kulturbedingungen im Reis vorkommende Unkräuter wie z.B. Sagittaria, Alisma, Eleocharis, Scirpus und Cyperus werden von den erfindungsgemäßen Wirkstoffen ebenfalls hervorragend bekämpft.

Werden die erfindungsgemäßen Verbindungen vor dem Keimen auf die Erdoberfläche appliziert, so wird entweder das Auflaufen der Unkrautkeimlinge vollständig verhindert oder die Unkräuter wachsen bis zum Keimblattstadium heran, stellen jedoch dann ihr Wachstum ein und sterben schließlich nach Ablauf von drei bis vier Wochen vollkommen ab.

Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt wird.

Obgleich die erfindungsgemäßen Verbindungen eine ausgezeichnete herbizide Aktivität gegenüber mono- und dikotylen Unkräutern aufweisen, werden Kulturpflanzen wirtschaftlich bedeutender Kulturen wie z.B. Weizen, Gerste, Roggen, Reis, Mais, Zuckerrübe, Baumwolle und Soja nur unwesentlich oder gar nicht geschädigt. Die vorliegenden Verbindungen eignen sich aus diesen Gründen sehr gut zur selektiven Bekämpfung von unerwünschtem Pflanzenwuchs in landwirtschaftlichen Nutzpflanzungen.

Darüber hinaus weisen die erfindungsgemäßen Substanzen hervorragende wachstumsregulatorische Eigenschaften bei Kulturpflanzen auf. Sie greifen regulierend in den pflanzeneigenen Stoffwechsel ein und können damit zur gezielten Beeinflussung von Pflanzeninhaltsstoffen und zur Ernteerleichterung, z.B. durch Auslösen von Desikkation und Wuchstauchung, eingesetzt werden. Desweiteren eignen sie sich auch zur generellen Steuerung und Hemmung von unerwünschtem vegetativem Wachstum, ohne dabei die Pflanzen abzutöten. Eine Hemmung des vegetativen Wachstums spielt bei vielen mono- und dikotylen Kulturen eine große Rolle, da das Lagern hierdurch verringert oder völlig verhindert werden kann.

Die erfindungsgemäßen Verbindungen können in Form von Spritzpulvern, emulgierbaren Konzentraten, versprühbaren Lösungen, Stäubemitteln oder Granulaten in den üblichen Zubereitungen angewendet werden. Gegenstand der Erfindung sind deshalb auch herbizide und pflanzenwachstumsregulierende Mittel, die Verbindungen der Formel (I) enthalten.

Die Verbindungen der Formel (I) können auf verschiedene Art formuliert werden, je nachdem welche biologischen und/oder chemisch-physikalischen Parameter vorgegeben sind. Als Formulierungsmöglichkeiten kommen beispielsweise in Frage: Spritzpulver (WP), wasserlösliche Pulver (SP), wasserlösliche Konzentrate, emulgierbare Konzentrate (EC), Emulsionen (EW), wie Öl-in-Wasser- und Wasser-in-Öl-Emulsionen, versprühbare Lösungen, Suspensionskonzentrate (SC), Dispersionen auf ÖI- oder Wasserbasis, ölmischbare Lösungen, Kapselsuspensionen (CS), Stäubemittel (DP), Beizmittel, Granulate für die Streu- und Bodenapplikation, Granulate (GR) in Form von Mikro-, Sprüh-, Aufzugs- und Adsorptionsgranulaten, wasserdispergierbare Granulate (WG), wasserlösliche Granulate (SG), ULV-Formulierungen, Mikrokapseln und Wachse.

Diese einzelnen Formulierungstypen sind im Prinzip bekannt und werden beispielsweise beschrieben in: Winnacker-Küchler, "Chemische Technologie", Band 7, C. Hauser Verlag München, 4. Aufl. 1986, Wade van Valkenburg, "Pesticide Formulations", Marcel Dekker, N.Y., 1973; K. Martens, "Spray Drying" Handbook, 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in: Watkins, "Handbook of Insecticide Dust Diluents and Carriers", 2nd Ed., Darland Books, Caldwell N.J., H.v. Olphen, "Introduction to Clay Colloid Chemistry"; 2nd Ed., J. Wiley & Sons, N.Y.; C. Marsden, "Solvents Guide"; 2nd Ed., Interscience, N.Y. 1963; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, wie z.B. Insektiziden, Akariziden, anderen Herbiziden, Fungiziden, Safenern, Düngemitteln und/oder Wachstumsregulatoren herstellen, z.B. in Form einer Fertigformulierung oder als Tankmix.

Spritzpulver sind in Wasser gleichmäßig dispergierbare Präparate, die neben dem Wirkstoff außer einem Verdünnungs- oder Inertstoff noch Tenside ionischer und/oder nichtionischer Art (Netzmittel, Dispergiermittel), z.B. polyoxyethylierte Alkylphenole, polyoxethylierte Fettalkohole, polyoxethylierte Fettamine, Fettalkoholpolyglykolethersulfate, Alkansulfonate, Alkylbenzolsulfonate, ligninsulfonsaures Natrium, 2,2'-dinaphthylmethan-6,6'-disulfonsaures Natrium, dibutylnaphthalin-sulfonsaures Natrium oder auch oleoylmethyltaurinsaures Natrium enthalten. Zur Herstellung der Spritzpulver werden die herbiziden Wirkstoffe beispielsweise in üblichen Apparaturen wie Hammermühlen,

Gebläsemühlen und Luftstrahlmühlen feingemahlen und gleichzeitig oder anschließend mit den Formulierungshilfsmitteln vermischt.

Emulgierbare Konzentrate werden durch Auflösen des Wirkstoffes in einem organischen Lösungsmittel z.B. Butanol, Cyclohexanon, Dimethylformamid, Xylol oder auch höhersiedenden Aromaten oder Kohlenwasserstoffen oder Mischungen der organischen Lösungsmittel unter Zusatz von einem oder mehreren Tensiden ionischer und/oder nichtionischer Art (Emulgatoren) hergestellt. Als Emulgatoren können beispielsweise verwendet werden: Alkylarylsulfonsaure Calcium-Salze wie Ca-dodecylbenzolsulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanester wie z.B. Sorbitanfettsäureester oder Polyoxethylensorbitanester wie z.B. Polyoxyethylensorbitanfettsäureester.

Stäubemittel erhält man durch Vermahlen des Wirkstoffes mit fein verteilten festen Stoffen, z.B. Talkum, natürlichen Tonen, wie Kaolin, Bentonit und Pyrophyllit, oder Diatomeenerde.

Suspensionskonzentrate können auf Wasser- oder Ölbasis sein. Sie können beispielsweise durch Naß-Vermahlung mittels handelsüblicher Perlmühlen und gegebenenfalls Zusatz von Tensiden, wie sie z.B. oben bei den anderen Formulierungstypen bereits aufgeführt sind, hergestellt werden.

Emulsionen, z.B. Öl-in-Wasser-Emulsionen (EW), lassen sich beispielsweise mittels Rührern, Kolloidmühlen und/oder statischen Mischern unter Verwendung von wäßrigen organischen Lösungsmitteln und gegebenenfalls Tensiden, wie sie z.B. oben bei den anderen Formulierungstypen bereits aufgeführt sind, herstellen.
Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

Wasserdispergierbare Granulate werden in der Regel nach den üblichen Verfahren wie Sprühtrocknung, Wirbelbett-Granulierung, Teller-Granulierung, Mischung mit Hochgeschwindigkeitsmischern und Extrusion ohne festes Inertmaterial hergestellt.

Die agrochemischen Zubereitungen enthalten in der Regel 0,1 bis 99 Gew.-%, insbesondere 0,1 bis 95 Gew.-%, Wirkstoff der Formel (I).

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 1 bis 90, vorzugsweise 5 bis 80 Gew.-% betragen. Staubförmige Formulierungen enthalten 1 bis 30, vorzugsweise meistens 5 bis 20 Gew.-% an Wirkstoff. versprühbare Lösungen etwa 0,05 bis 80, vorzugsweise 2 bis 50 Gew.-% Wirkstoff. Bei wasserdispergierbaren Granulaten hängt der Wirkstoffgehalt zum Teil davon ab, ob die wirksame Verbindung flüssig oder fest vorliegt und welche Granulierhilfsmittel, Füllstoffe usw. verwendet werden. Bei den in Wasser dispergierbaren Granulaten liegt der Gehalt an Wirkstoff beispielsweise zwischen 1 und 95 Gew.-%, vorzugsweise zwischen 10 und 80 Gew.-%.

Daneben enthalten die genannten Wirkstofformulierungen gegebenenfalls die jeweils üblichen Haft-, Netz-, Dispergier-, Emulgier-, Penetrations-, Konservierungs-, Frostschutz- und Lösungsmittel, Füll-, Träger- und Farbstoffe, Entschäumer, Verdunstungshemmer und den pH-Wert und die Viskosität beeinflussende Mittel.

Als Kombinationspartner für die erfindungsgemäßen Wirkstoffe in Mischungsformulierungen oder im Tank-Mix sind beispielsweise bekannte Wirkstoffe einsetzbar, wie sie in z.B. in Weed Research 26, 441-445 (1986), oder "The Pesticide Manual", 10th edition, The British Crop Protection Council and the Royal Society of Chemistry, 1994, England, und dort zitierter Literatur beschrieben sind. Als literaturbekannte Herbizide, die mit den Verbindungen der Formel (I) kombiniert werden können, sind z.B. folgende Wirkstoffe zu nennen (Anmerkung: Die Verbindungen sind entweder mit dem "common name" nach der International Organization for Standardization (ISO) oder mit dem chemischen Namen, ggf. zusammen mit einer üblichen Codenummer bezeichnet):
acetochlor; acifluorfen; aclonifen; AKH 7088, d.h. [[[1-[5-[2-Chloro-4-(trifluoromethyl)-phenoxy]-2-nitrophenyl]-2-methoxyethylidene]-amino]-oxy]-essigsäure und -essigsäuremethylester; alachlor; alloxydim; ametryn; amidosulfuron; amitrol; AMS, d.h. Ammoniumsulfamat; anilofos; asulam; atrazin; aziprotryn; barban; BAS 516 H, d.h. 5-Fluor-2-phenyl-4H-3,1-benzoxazin-4-on; benazolin; benfluralin; benfuresate; bensulfuron-methyl; bensulide; bentazone; benzofenap; benzofluor; benzoylprop-ethyl; benzthiazuron; bialaphos; bifenox; bromacil; bromobutide; bromofenoxim; bromoxynil; bromuron; buminafos; busoxinone; butachlor; butamifos; butenachlor; buthidazole; butralin; butylate; carbetamide; CDAA, d.h. 2-Chlor-N,N-di-2-propenylacetamid; CDEC, d.h. Diethyldithiocarbaminsäure-2-chlorallylester; CGA 184927, d.h. 2-[4-[(5-Chlor-3-fluor-2-pyridinyl)-oxy]-phenoxy]-propansäure und 2-propynylester; chlomethoxyfen; chloramben; chlorazifop-butyl, pirifenop-butyl; chlorbromuron; chlorbufam; chlorfenac; chlorflurecol-methyl; chloridazon; chlorimuron ethyl; chlornitrofen; chlorotoluron; chloroxuron; chlorpropham; chlorsulfuron; chlorthal-dimethyl; chlorthiamid; cinmethylin; cinosulfuron; clethodim; clomazone; clomeprop; cloproxydim; clopyralid; cyanazine; cycloate; cycloxydim; cycluron; cyperquat; cyprazine; cyprazole; 2,4-DB; dalapon; desmedipham; desmetryn; di-allate; dicamba; dichlobenil; dichlorprop; diclofop-methyl; diethatyl; difenoxuron; difenzoquat; diflufenican; dimefuron; dimethachlor; dimethametryn; dimethazone, clomazon; dimethipin; dimetrasulfuron, cinosulfuron; dinitramine; dinoseb; dinoterb; diphenamid; dipropetryn; diquat; dithiopyr; diuron; DNOC; eglinazine-ethyl; EL 177, d.h. 5-Cyano-1-(1,1-dimethylethyl)-N-methyl-3H-pyrazole-4-carboxamid; endothal; EPTC; esprocarb; ethalfluralin; ethametsulfuron-methyl; ethidimuron; ethiozin; ethofumesate; F5231, d.h. N-[2-Chlor-4-fluor-5-[4-(3-fluorpropyl)-4,5-dihydro-5-oxo-1 H-tetrazol-1-yl]-phenyl]-ethansulfonamid; F6285, d.h. 1-[5-(N-Methylsulfonyl)-amino-2,4-dichlorophenyl]-3-methyl-4-difluoromethyl-1,2,4-triazol-5-on; fenoprop; fenoxan, s. clomazon; fenoxaprop-ethyl; fenuron; flamprop-methyl; flazasulfuron; fluazifop und dessen Esterderivate; fluchloralin; flumetsulam; N-[2,6-Difluorphenyl]-5-methyl-(1,2,4)-triazolo[1,5a]pyrimidin-2- sulfonamid; flumeturon; flumipropyn; fluorodifen; fluoroglycofen-ethyl; fluridone; flurochloridone; fluroxypyr; flurtamone; fomesafen; fosamine; furyloxyfen; glufosinate; glyphosate; halosafen; haloxyfop und dessen Esterderivate; hexazinone; Hw 52, d.h. N-(2,3-Dichlorphenyl)-4-(ethoxymethoxy)-benzamid; imazamethabenz-methyl imazapyr; imazaquin; imazethamethapyr; imazethapyr; imazosulfuron; ioxynil; isocarbamid; isopropalin; isoproturon; isouron; isoxaben; isoxapyrifop; karbutilate; lactofen; lenacil; linuron; MCPA; MCPB; mecoprop; mefenacet; mefluidid; metamitron; metazachlor; methabenzthiazuron; metham; methazole; methoxyphenone; methyldymron; metobromuron; metolachlor; metoxuron; metribuzin; metsulfuron-methyl; MH; molinate; monalide; monocarbamide dihydrogensulfate; monolinuron; monuron; MT 128, d.h. 6-Chlor-N-(3-chlor-2-propenyl)-5-methyl-N-phenyl-3-pyridazinamin; MT 5950, d.h. N-[3-Chlor-4-(1 -methylethyl)-phenyl]-2-methylpentanamid; naproanilide; napropamide; naptalam; NC 310, d.h. 4-(2,4-dichlorbenzoyl)-1-methyl-5-benzyloxypyrazol; neburon; nicosulfuron; nipyraclophen; nitralin; nitrofen; nitrofluorfen; norflurazon; orbencarb; oryzalin; oxadiazon; oxyfluorfen; paraquat; pebulate; pendimethalin; perfluidone; phenmedipham; phenisopham; phenmedipham; picloram; piperophos; piributicarb; pirifenop-butyl; pretilachlor; primisulfuron-methyl; procyazine; prodiamine; profluralin; proglinazine-ethyl; prometon; prometryn; propachlor; propanil; propaquizafop und dessen Esterderivate; propazine; propham; propyzamide; prosulfalin; prosulfocarb; prynachlor; pyrazolinate; pyrazon; pyrazosulfuron-ethyl; pyrazoxyfen; pyridate; quinclorac; quinmerac; quinofop und dessen Esterderivate, quizalofop und dessen Esterderivate; quizalofop-ethyl; quizalofop-p-tefuryl; renriduron; dymron; S 275, d.h. 2-[4-Chlor-2-fluor-5-(2-propynyloxy)-phenyl]-4,5,6,7-tetrahydro-2H-indazol; S 482, d.h. 2-[7-Fluor-3,4-dihydro-3-oxo-4-(2-propynyl)-2H-1,4-benzoxazin-6-yl]-4,5,6,7-tetrahydro-1H-isoindol-1,3(2H)-dion; secbumeton; sethoxydim; siduron; simazine; simetryn; SN 106279, d.h. 2-[[7-[2-Chlor-4-(trifluor-methyl)-phenoxy]-2-naphthalenyl]-oxy]-propansäure und -methylester; sulfometuron-methyl; sulfazuron; flazasulfuron; TCA; tebutam; tebuthiuron; terbacil; terbucarb; terbuchlor; terbumeton; terbuthylazine; terbutryn; TFH 450, d.h. N,N-Diethyl-3-[(2-ethyl-6-methylphenyl)-sulfonyl]-1H-1,2,4-triazol-1-carboxamid; thiazafluron; thifensulfuron-methyl; thiobencarb; tiocarbazil; tralkoxydim; tri-allate; triasulfuron; triazofenamide; tribenuron-methyl; triclopyr; tridiphane; trietazine; trifluralin; trimeturon; vernolate; WL 110547, d.h. 5-Phenoxy- 1-[3-(trifluormethyl)-phenyl]- 1H-tetrazol.

Zur Anwendung werden die in handelsüblicher Form vorliegenden Formulierungen gegebenenfalls in üblicher Weise verdünnt, z.B. bei Spritzpulvern, emulgierbaren Konzentraten, Dispersionen und wasserdispergierbaren Granulaten mittels Wasser, und anschließend auf die Pflanzen, Pflanzenteile oder den landwirtschaftlich oder industriell genützten Boden, auf dem die Pflanzen stehen oder in dem sie heranwachsen oder als Saat vorliegen, appliziert. Staubförmige Zubereitungen, Boden- bzw. Streugranulate sowie versprühbare Lösungen werden vor der Anwendung üblicherweise nicht mehr mit weiteren inerten Stoffen verdünnt.

Mit den äußeren Bedingungen wie Temperatur, Feuchtigkeit, der Art des verwendeten Herbizids, u.a. variiert die erforderliche Aufwandmenge der Verbindungen der Formel (1). Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.
A. Chemische Beispiele
   A.1 N-[(4,6-Dimethoxypyrimidin-2-yl)-aminocarbonyl]-5-formylamino-2-methoxycarbonylbenzolsulfonamid (Tabelle 1, Bsp. 1)
      1,30 g 5-Formylamino-2-methoxycarbonyl-benzolsulfonamid und 1,40 g 4,6-Dimethoxy-2-phenoxycarbonylamino-pyrimidin werden in 15 ml Acetonitril suspendiert und bei 0°C mit 2,3 ml DBU versetzt. Nach 18 Stunden wird die Reaktionslösung eingeengt. Der Rückstand wird in Wasser aufgenommen und mit Diethylether gewaschen. Nach vorsichtigem Ansäuern der wäßrigen Phase mit konzentrierter Salzsäure (pH = 1 bis 2) bei 0°C wird der abgeschiedene Sulfonylharnstoff mit Methanol und Diisopropylether verrührt. Man erhält so 0,91 g des gewünschten Sulfonylharnstoffes; Massenspektrum (Cl):(M + 1) = 440.
   A.2 N-[(4,6-Dimethoxypyrimidin-2-yl)-aminocarbonyl]-5-formylamino-2-n-propoxycarbonylbenzolsulfonamid (Tabelle 1, Bsp. 15)
      Analog dem chemischen Beispiel A.1 werden 1,50 g 5-Formylamino-2-propoxycarbonylbenzolsulfonamid, 1,59 g 4,6-Dimethoxy-2-phenoxycarbonylamino-pyrimidin und 1,8 ml DBU in 30 ml Acetonitril umgesetzt; Ausbeute: 1,9 g, Fp.: 151 bis 153°C (Zers.); Massenspektrum (Cl):(M + 1) = 468
   A.3. N-[(4,6-Dimethoxypyrimidin-2-yl)-aminocarbonyl]-5-formylamino-2-ethoxycarbonylbenzolsulfonamid (Tabelle 1, Bsp. 9)
      Analog dem chemischen Beispiel A.1 werden 1,00 g 5-Formylamino-2-ethoxycarbonylbenzolsulfonamid, 1,11 g 4,6-Dimethoxy-2-phenoxycarbonylamino-pyrimidin und 0,82 ml DBU in Acetonitril umgesetzt; Ausbeute: 1,00 g, Fp.: 113 bis 118°C (Zers.), Massenspektrum (Cl):(M +1) = 454

   Die in der nachfolgenden Tabelle 1 beschriebenen Verbindungen erhält man auf analoge Weise.
   Folgende Abkürzungen werden in der Tabelle 1 verwendet:
   - Nr. =: Beispielnummer
   - Fp. =: Festpunkt (Schmelzpunkt)
   - Me =: Methyl
   - Et =: Ethyl
   - Pr =: ⁿPr = n-Propyl
   - ⁱPr =: i-Propyl
   - ^{c}Pr =: Cyclopropyl
   - (Z) =: Zersetzung 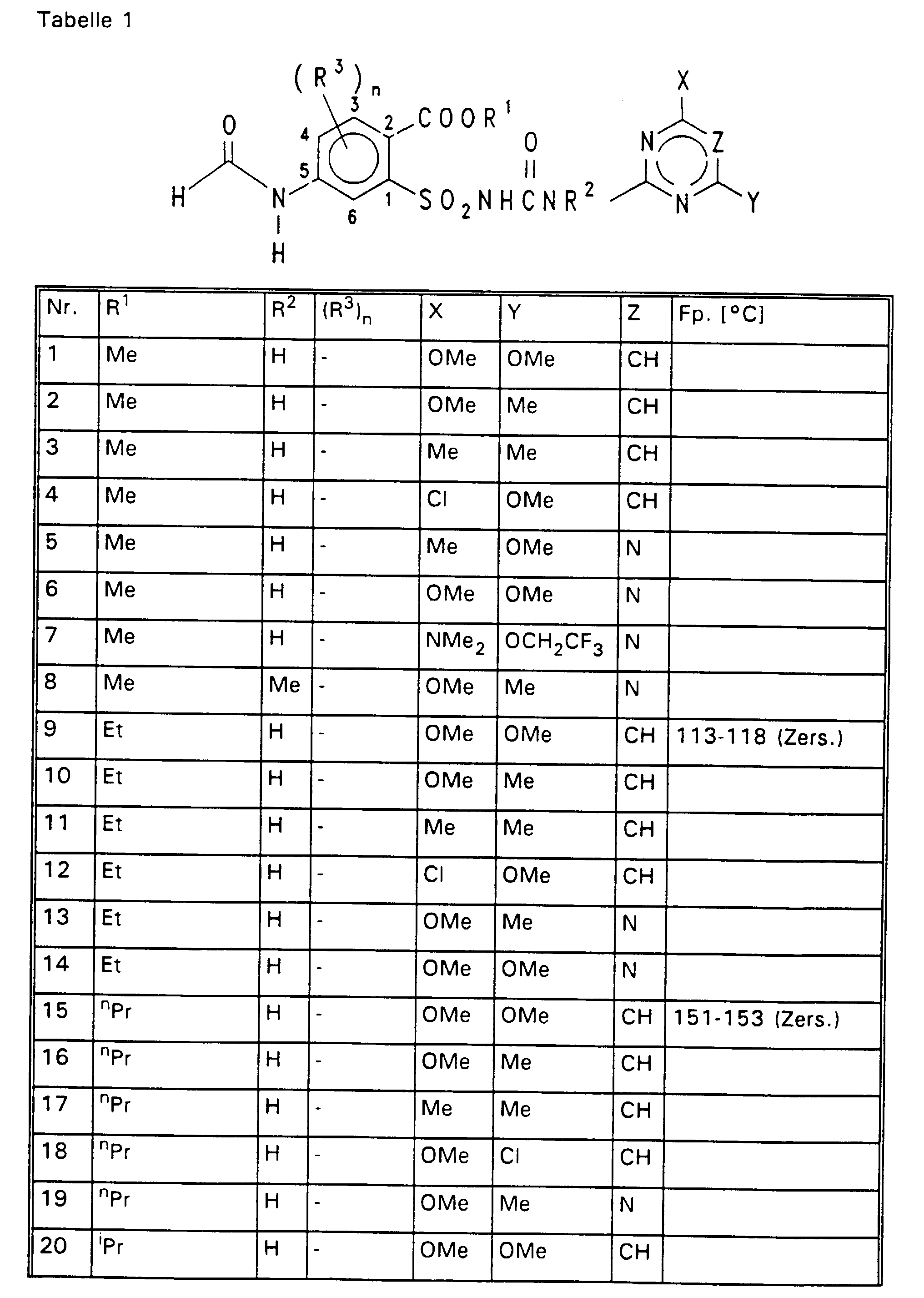 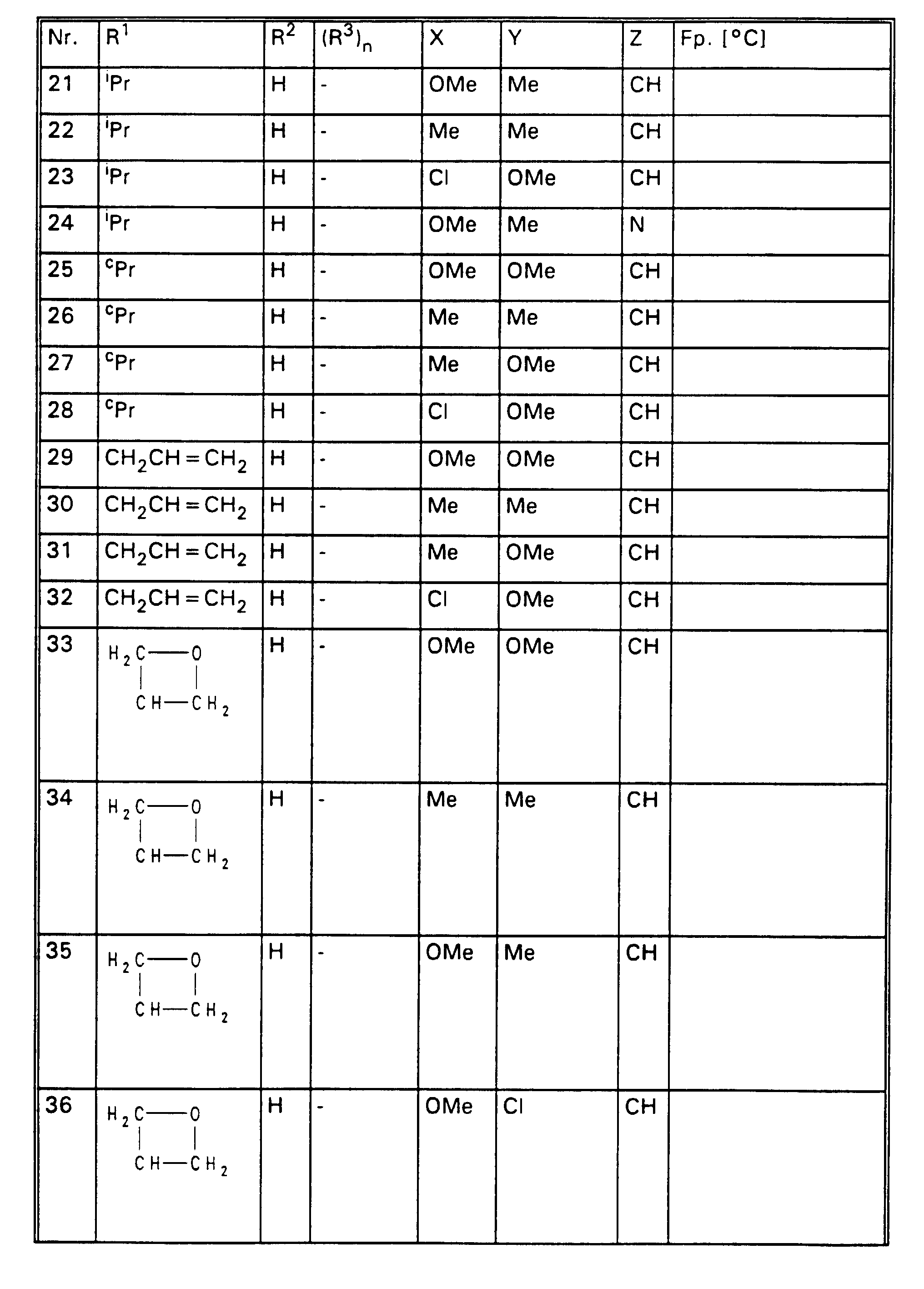 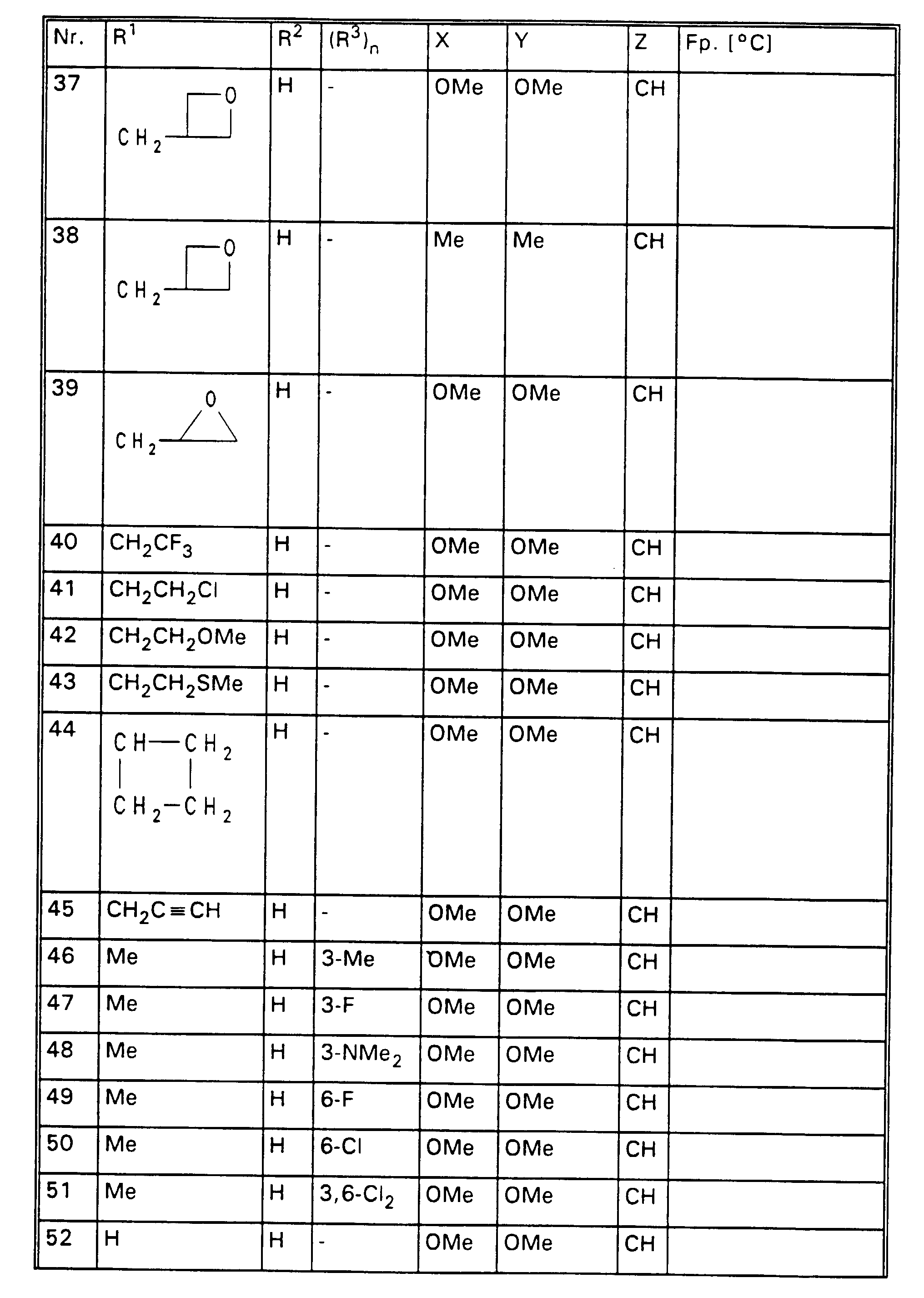 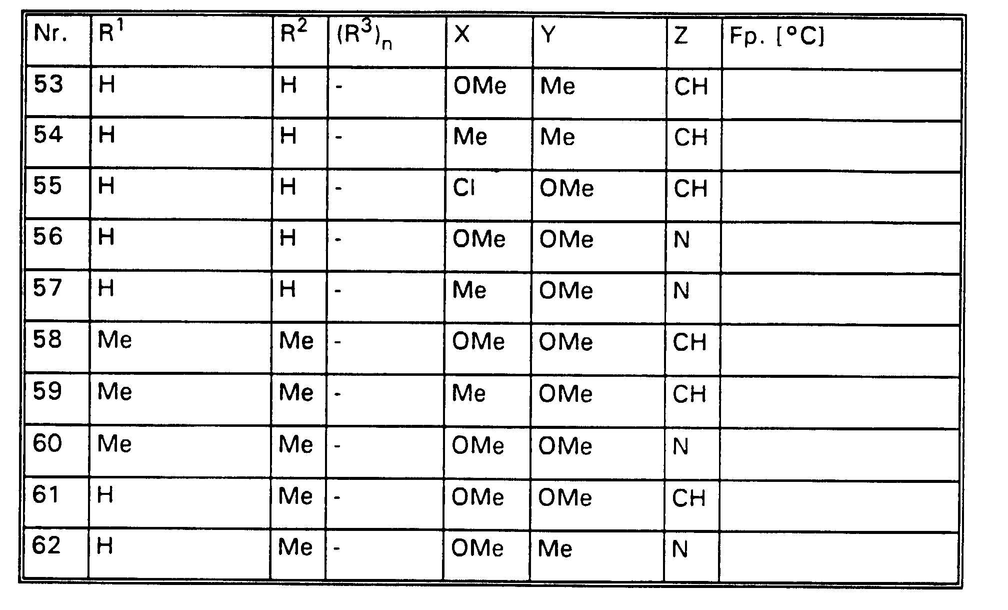
B. Formulierungsbeispiele
   a) Ein Stäubemittel wird erhalten, indem man 10 Gew.-Teile einer Verbindung der Formel (I) und 90 Gew.-Teile Talkum als Inertstoff mischt und in einer Schlagmühle zerkleinert.
   b) Ein in Wasser leicht dispergierbares, benetzbares Pulver wird erhalten, indem man 25 Gewichtsteile einer Verbindung der Formel (I), 64 Gewichtsteile kaolinhaltigen Quarz als Inertstoff, 10 Gewichtsteile ligninsulfonsaures Kalium und 1 Gewichtsteil oleoylmethyltaurinsaures Natrium als Netz- und Dispergiermittel mischt und in einer Stiftmühle mahlt.
   c) Ein in Wasser leicht dispergierbares Dispersionskonzentrat wird erhalten, indem man 20 Gewichtsteile einer Verbindung der Formel (I) mit 6 Gewichtsteilen Alkylphenolpolyglykolether (^{®} Triton X 207), 3 Gewichtsteilen Isotridecanolpolyglykolether (8 EO) und 71 Gewichtsteilen paraffinischem Mineralöl (Siedebereich z.B. ca. 255 bis über 277°C) mischt und in einer Reibkugelmühle auf eine Feinheit von unter 5 Mikron vermahlt.
   d) Ein emulgierbares Konzentrat wird erhalten aus 15 Gewichtsteilen einer Verbindung der Formel (I), 75 Gewichtsteilen Cyclohexanon als Lösungsmittel und 10 Gewichtsteilen oxethyliertes Nonylphenol als Emulgator.
   e) Ein in Wasser dispergierbares Granulat wird erhalten indem man
      75 Gewichtsteile einer Verbindung der Formel (I),
      10 Gewichtsteile ligninsulfonsaures Calcium,
      5 Gewichtsteile Natriumlaurylsulfat,
      3 Gewichtsteile Polyvinylalkohol und
      7 Gewichtsteile Kaolin
      mischt, auf einer Stiftmühle mahlt und das Pulver in einem Wirbelbett durch Aufsprühen von Wasser als Granulierflüssigkeit granuliert.
   f) Ein in Wasser dispergierbares Granulat wird auch erhalten, indem man
      25 Gewichtsteile einer Verbindung der Formel (I),
      5 Gewichtsteile 2,2'-dinaphthylmethan-6,6'-disulfonsaures Natrium
      2 Gewichtsteile oleoylmethyltaurinsaures Natrium,
      1 Gewichtsteil Polyvinylalkohol,
      17 Gewichtsteile Calciumcarbonat und
      50 Gewichtsteile Wasser
      auf einer Kolloidmühle homogenisiert und vorzerkleinert, anschließend auf einer Perlmühle mahlt und die so erhaltene Suspension in einem Sprühturm mittels einer Einstoffdüse zerstäubt und trocknet.
C. Biologische Beispiele
   1. Unkrautwirkung im Vorauflauf
      Samen bzw. Rhizomstücke von mono- und dikotylen Unkraufpflanzen werden in Plastiktöpfen in sandiger Lehmerde ausgelegt und mit Erde abgedeckt. Die in Form von benetzbaren Pulvern oder Emulsionskonzentraten formulierten erfindungsgemäßen Verbindungen werden dann als wäßrige Suspension bzw. Emulsion mit einer Wasseraufwandmenge von umgerechnet 600 bis 800 I/ha in unterschiedlichen Dosierungen auf die Oberfläche der Abdeckerde appliziert.
      Nach der Behandlung werden die Töpfe im Gewächshaus aufgestellt und unter guten Wachstumsbedingungen für die Unkräuter gehalten. Die optische Bonitur der Pflanzen- bzw. der Auflaufschäden erfolgt nach dem Auflaufen der Versuchspflanzen nach einer Versuchszeit von 3 bis 4 Wochen im Vergleich zu unbehandelten Kontrollen. Wie die Testergebnisse zeigen, weisen die erfindungsgemäßen Verbindungen eine gute herbizide Vorauflaufwirksamkeit gegen ein breites Spektrum von Ungräsern und Unkräutern auf. Beispielsweise haben die Verbindungen der Beispiele 1, 9 und 15 aus der Tabelle 1 sehr gute herbizide Wirkung gegen Schadpflanzen wie Alopecurus myosuroides, Sinapis alba, Chrysanthemum segetum, Avena sativa, Stellaria media, Echinochlora crus-galli und Lolium multiflorum im Vorauflaufverfahren bei einer Aufwandmenge von 0,3 kg und weniger Aktivsubstanz pro Hektar.
   2. Unkrautwirkung im Nachauflauf
      Samen bzw. Rhizomstücke von mono- und dikotylen Unkräutern werden in Plastiktöpfen in sandigem Lehmboden ausgelegt, mit Erde abgedeckt und im Gewächshaus unter guten Wachstumsbedingungen angezogen. Drei Wochen nach der Aussaat werden die Versuchspflanzen im Dreiblattstadium behandelt.
      Die als Spritzpulver bzw. als Emulsionskonzentrate formulierten erfindungsgemäßen Verbindungen werden in verschiedenen Dosierungen mit einer Wasseraufwandmenge von umgerechnet 600 bis 800 I/ha auf die grünen Pflanzenteile gesprüht und nach ca. 3 bis 4 Wochen Standzeit der Versuchspflanzen im Gewächshaus unter optimalen Wachstumsbedingungen die Wirkung der Präparate optisch im Vergleich zu unbehandelten Kontrollen bonitiert. Die erfindungsgemäßen Mittel weisen auch im Nachauflauf eine gute herbizide Wirksamkeit gegen ein breites Spektrum wirtschaftlich wichtiger Ungräser und Unkräuter auf. Beispielsweise haben die Verbindungen der Beispiele 1, 9 und 15 aus der Tabelle 1 sehr gute herbizide Wirkung gegen Schadpflanzen wie Alopecurus myosuroides, Sinapis alba, Stellaria media, Echinochloa crus-galli, Lolium multiflorum, Chrysanthemum segetum und Avena sativa im Nachauflaufverfahren bei einer Aufwandmenge von 0,3 kg und weniger Aktivsubstanz pro Hektar.
   3. Kulturpflanzenverträglichkeit
      In weiteren Versuchen im Gewächshaus werden Samen einer größeren Anzahl von Kulturpflanzen und Unkräutern in sandigem Lehmboden ausgelegt und mit Erde abgedeckt. Ein Teil der Töpfe wird sofort wie unter Abschnitt 1 beschrieben behandelt, die übrigen im Gewächshaus aufgestellt, bis die Pflanzen zwei bis drei echte Blätter entwickelt haben und dann wie unter Abschnitt 2 beschrieben mit den erfindungsgemäßen Substanzen der Formel (I) in unterschiedlichen Dosierungen besprüht. Vier bis fünf Wochen nach der Applikation und Standzeit im Gewächshaus wird mittels optischer Bonitur festgestellt, daß die erfindungsgemäßen Verbindungen zweikeimblättrige Kulturen wie z.B. Soja, Baumwolle, Raps, Zuckerrüben und Kartoffeln im Vor- und Nachauflaufverfahren selbst bei hohen Wirkstoffdosierungen ungeschädigt lassen. Einige Substanzen schonen darüber hinaus auch Gramineen-Kulturen wie z.B. Gerste, Weizen, Roggen, Sorghum-Hirsen, Mais oder Reis. Die Verbindungen der Formel (I) weisen somit eine hohe Selektivität bei Anwendung zur Bekämpfung von unerwünschten Pflanzenwuchs in landwirtschaftlichen Kulturen auf.

## Patentansprüche

1. Verbindungen der Formel (I), 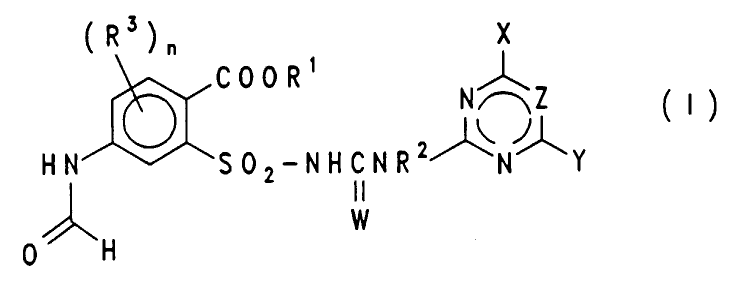 worin
R¹ H, einen substituierten oder unsubstituierten Kohlenwasserstoffrest oder einen unsubstituierten oder substituierten heterocyclischen Rest,
R² H, (C₁-C₆)-Alkyl oder (C₁-C₆)-Alkoxy,
R³ Halogen, (C₁-C₆₎-Alkyl, (C₁-C₆)-Alkoxy, (C₁-C₆)-Haloalkyl, (C₁-C₆)-Haloalkoxy, NO₂, CN, NH₂, (C₁-C₄)-Mono- oder Dialkylamino, und zwar jeweils unabhängig von anderen Resten R³, wenn n 2 oder 3 ist,
n 0, 1, 2 oder 3,
W ein Sauerstoffatom oder ein Schwefelatom,
X, Y unabhängig voneinander Halogen, (C₁-C₆)-Alkvl, (C₁-C₆)-Alkoxy, (C₁-C₆)-Alkylthio, wobei jeder der drei letztgenannten Reste unsubstituiert oder durch einen oder mehrere Reste aus der Gruppe Halogen, (C₁-C₄)-Alkoxy und (C₁-C₄)-Alkylthio substituiert ist, oder (C₃-C₆)-Cycloalkyl, (C₂-C₆)-Alkenyl, (C₂-C₆)-Alkinyl, (C₂-C₆)-Alkenyloxy, (C₂-C₆)-Alkinyloxy, Mono- oder Di-[(C₁-C₄)-alkyl]-amino und
Z CH oder N bedeuten.

2. Verbindungen der Formel (I) nach Anspruch 1, dadurch gekennzeichnet, daß
R¹ H, (C₁-C₆)-Alkyl, (C₂-C₆)-Alkenyl, (C₂-C₆)-Alkinyl, (C₃-C₆)-Cycloalkyl, wobei die vier letztgenannten Reste unsubstituiert oder durch einen oder mehrere Reste aus der Gruppe Halogen, CN, (C₁-C₃)-Alkoxy, (C₁-C₃)-Haloalkoxy, (C₃-C₆)-Cycloalkyl, (C₁-C₃)-Alkylthio, (C₁-C₃)-Alkylsulfinyl, (C₁-C₃)-Alkylsulfonyl, unsubstituiertes Phenyl, substituiertes Phenyl, unsubstituierter heterocyclischer Rest und substituierter heterocyclischer Rest und im Falle cyclischer Reste auch (C₁-C₄)-Alkyl substituiert sind, oder einen heterocyclischen Rest mit 3, 4, 5, 6 oder 7 Ringatomen und ein oder mehreren Atomen aus der Gruppe O, N und S als Heteroringatom, wobei der Rest unsubstituiert oder durch einen oder mehrere Reste aus der Gruppe Halogen, (C₁-C₃)-Alkyl und (C₁-C₃)-Haloalkyl substituiert ist,
R² H, (C₁-C₃)-Alkoxy oder (C₁-C₃)-Alkyl,
R³ Halogen, (C₁-C₃)-Alkyl, (C₁-C₃)-Alkoxy, (C₁-C₃)-Haloalkyl, (C₁-C₃)-Haloalkoxy, NO₂, CN, NH₂, NHCH₃, N(CH₃)₂,
n 0, 1, 2 oder 3,
W O oder S,
X, Y unabhängig voneinander Halogen, (C₁-C₄)-Alkyl, (C₁-C₄)-Alkoxy, (C₁-C₄)-Alkylthio, wobei jeder der drei letztgenannten Reste unsubstituiert oder durch einen oder mehrere Reste aus der Gruppe Halogen, (C₁-C₄)-Alkoxy und (C₁-C₄)-Alkylthio substituiert ist, oder (C₃-C₆)-Cycloalkyl, (C₂-C₄)- Alkenyl, (C₂-C₄)-Alkinyl, (C₂-C₄)-Alkenyloxy, (C₂-C₄)-Alkinyloxy, Mono- oder Di-[(C₁-C₂)-alkyl]-amino und
Z CH oder N bedeuten.

3. Verbindungen der Formel (I) nach Anspruch 1 oder 2, dadurch gekennzeichnet, daß
R¹ (C₁-C₆)-Alkyl, (C₂-C₆)-Alkenyl, (C₂-C₆)-Alkinyl, (C₃-C₆)-Cycloalkyl, (C₄-C₈)-Cycloalkylalkyl, Phenyl-(C₁-C₆)-alkyl, wobei jeder der sechs letztgenannten Reste unsubstituiert oder durch einen oder mehrere Reste aus der Gruppe Halogen, CN, OCH₃, OC₂H₅, OCF₃, SO₂CH₃ und im Falle cyclischer Reste auch (C₁-C₃)-Alkyl substituiert ist,
oder einen Rest der Formeln A₁ bis A₇, 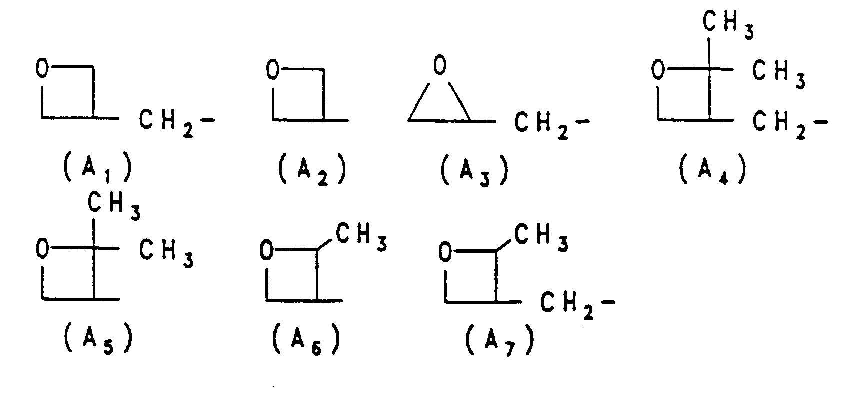
R² H oder CH₃,
R³ Halogen, CH₃, C₂H₅, OCH₃, OC₂H₅, CF₃, CCl₃, OCF₃, OCHF₂ oder N(CH₃)₂, und
n 0, 1 oder 2
bedeuten.

4. Verfahren zur Herstellung von Verbindungen der Formel (I) gemäß Anspruch 1, dadurch gekennzeichnet, daß man
a) eine Verbindung der Formel (II) 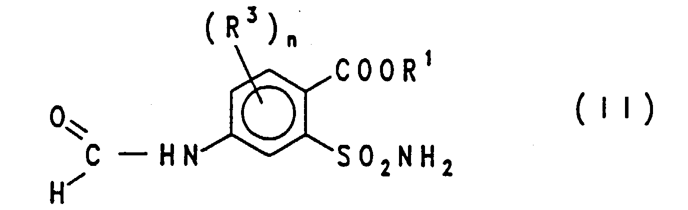 mit einem heterocyclischen Carbamat der Formel (III), 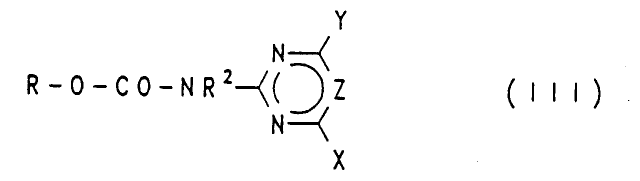 worin R unsubstituiertes oder substituiertes Phenyl bedeutet, umsetzt oder
b) ein Sulfochlorid der Formel (IV) 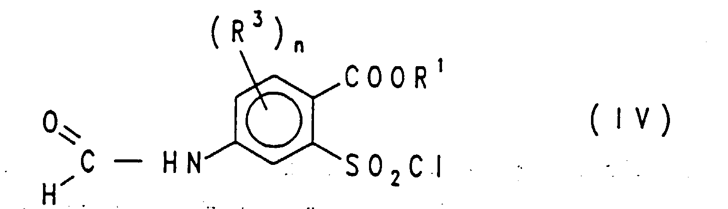 mit einem heterocyclischen Amin der Formel (V) 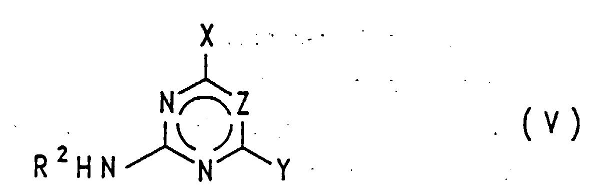 in Gegenwart eines Cyanats umsetzt oder
c) einen Sulfonylharnstoff der Formel (VI) 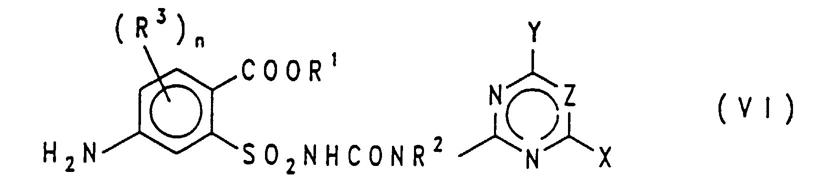 formyliert oder
d) ein Sulfonamid der Formel (II) mit einem (Thio)lsocyanat der Formel (VII) in Gegenwart einer geeigneten Base umsetzt, 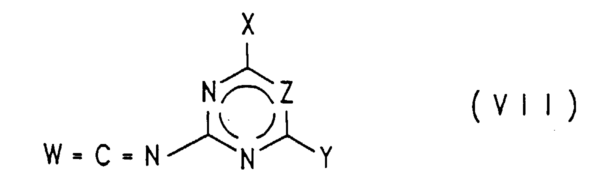
wobei in den obigen Formeln (II) bis (VII) die Reste R¹, R², R³, W, X, Y und Z sowie der Index n wie in Formel (I) definiert sind und in den Verfahrensvarianten a), b) und c) zunächst Verbindungen erhalten werden, in denen W ein Sauerstoffatom bedeutet.

5. Herbizides oder pflanzenwachstumsregulierendes Mittel, dadurch gekennzeichnet, daß es mindestens eine Verbindung der Formel (I) nach einem der Ansprüche 1 bis 3 und im Pflanzenschutz übliche Formulierungshilfsmittel enthält.

6. Verfahren zur Bekämpfung von Schadpflanzen oder zur Wachstumsregulierung von Pflanzen, dadurch gekennzeichnet, daß man ein wirksame Menge von mindestens einer Verbindung der Formel (I) nach einem der Ansprüche 1 bis 3 auf die Schadpflanzen bzw. Pflanzen, deren Pflanzensamen oder die Fläche, auf der die Pflanzen wachsen, appliziert.

7. Verwendung der Verbindungen der Formel (I) nach einem der Ansprüche 1 bis 3 als Herbizide oder Pflanzenwachstumsregulatoren.

8. Verbindungen der Formel (II), wie sie in Anspruch 4 definiert sind.

## Claims

1. A compound of the formula (I) 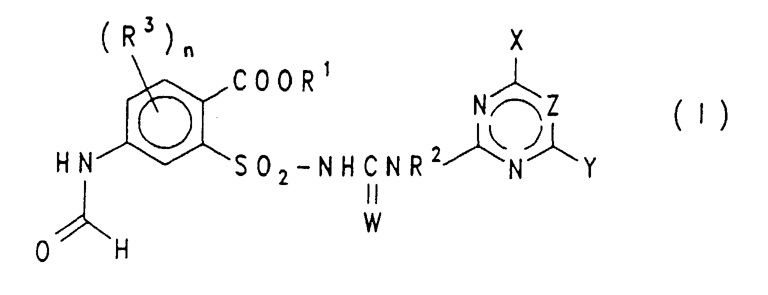 in which
R¹ is H, a substituted or unsubstituted hydrocarbon radical or an unsubstituted or substituted heterocyclic radical,
R² is H, (C₁-C₆)-alkyl or (C₁-C₆)-alkoxy,
R³ is halogen, (C₁-C₆)-alkyl, (C₁-C₆)-alkoxy, (C₁-C₆)-haloalkyl, (C₁-C₆)-haloalkoxy, NO₂, CN, NH₂ or (C₁-C₄)-mono- or dialkylamino, each independently of other radicals R³ if n is 2 or 3,
n is 0, 1, 2 or 3,
W is an oxygen atom or a sulfur atom,
X and Y independently of one another are halogen, (C₁-C₆)-alkyl, (C₁-C₆)-alkoxy or (C₁-C₆)-alkyl-thio, each of the three last-mentioned radicals being unsubstituted or substituted by one or more radicals selected from the group consisting of halogen, (C₁-C₄)-alkoxy and (C₁-C₄)-alkylthio, or are (C₃-C₆)-cycloalkyl, (C₂-C₆)-alkenyl, (C₂-C₆)-alkynyl, (C₂-C₆)-alkenyloxy, (C₂-C₆)-alkynyloxy or mono- or di[(C₁-C₄)-alkyl]amino, and
Z is CH or N.

2. A compound of the formula (I) as claimed in claim 1, wherein
R¹ is H, (C₁-C₆)-alkyl, (C₂-C₆)-alkenyl, (C₂-C₆)-alkynyl or (C₃-C₆)-cycloalkyl, the four last-mentioned radicals being unsubstituted or substituted by one or more radicals selected from the group consisting of halogen, CN, (C₁-C₃)-alkoxy, (C₁-C₃)-haloalkoxy, (C₃-C₆)-cycloalkyl, (C₁-C₃)-alkylthio, (C₁-C₃)-alkylsulfinyl, (C₁-C₃) -alkylsulfonyl, unsubstituted phenyl, substituted phenyl, unsubstituted heterocyclic radical and substituted heterocyclic radical, and, in the case of cyclic radicals, also (C₁-C₄)-alkyl,
or is a heterocyclic radical having 3, 4, 5, 6 or 7 ring atoms and one or more atoms selected from the group consisting of O, N and S as a heterocyclic ring atom, the radical being unsubstituted or substituted by one or more radicals selected from the group consisting of halogen, (C₁-C₃)-alkyl and (C₁-C₃)-haloalkyl,
R² is H, (C₁-C₃)-alkoxy or (C₁-C₃)-alkyl,
R³ is halogen, (C₁-C₃)-alkyl, (C₁-C₃)-alkoxy, (C₁-C₃)-haloalkyl, (C₁-C₃)-haloalkoxy, NO₂, CN, NH₂, NHCH₃, N(CH₃)₂,
n is 0, 1, 2 or 3,
W is O or S,
X and Y independently of one another are halogen, (C₁-C₄)-alkyl, (C₁-C₄)-alkoxy or (C₁-C₄)-alkylthio, each of the three last-mentioned radicals being unsubstituted or substituted by one or more radicals selected from the group consisting of halogen, (C₁-C₄)-alkoxy and (C₁-C₄)-alkylthio, or are (C₃-C₆)-cycloalkyl, (C₂-C₄)-alkenyl, (C₂-C₄)-alkynyl, (C₂-C₄)-alkenyloxy, (C₂-C₄)-alkynyloxy or mono- or di[(C₁-C₂)-alkyl]amino, and
Z is CH or N.

3. A compound of the formula (I) as claimed in claim 1 or 2, wherein
R¹ is (C₁-C₆)-alkyl, (C₂-C₆)-alkenyl, (C₂-C₆)-alkynyl, (C₃-C₆)-cycloalkyl, (C₄-C₈)-cyclo-alkylalkyl or phenyl(C₁-C₆)-alkyl, each of the six last-mentioned radicals being unsubstituted or substituted by one or more radicals selected from the group consisting of halogen, CN, OCH₃, OC₂H₅, OCF₃ and SO₂CH₃ and, in the case of cyclic radicals, also (C₁-C₃)-alkyl, or is a radical of the formulae A₁ to A₇ 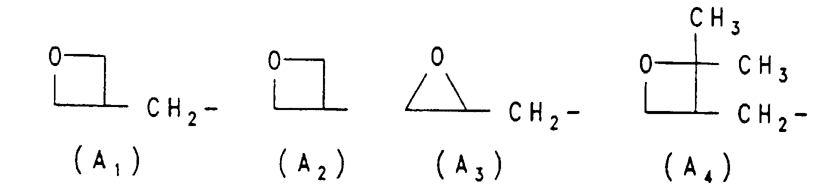 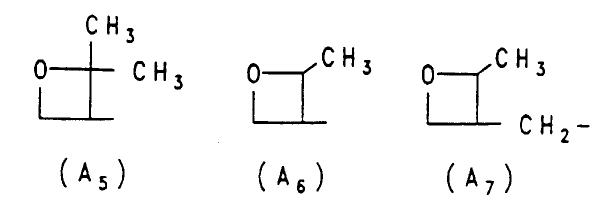
R² is H or CH₃,
R³ is halogen, CH₃, C₂H₅, OCH₃, OC₂H₅, CF₃, CCl₃, OCF₃, OCHF₂ or N(CH₃)₂, and
n is 0, 1 or 2.

4. A process for the preparation of compounds of the formula (I) as claimed in claim 1, which comprises
a) reacting a compound of the formula (II) 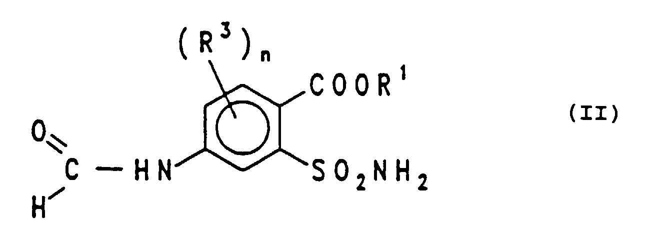 with a heterocyclic carbamate of the formula (III) 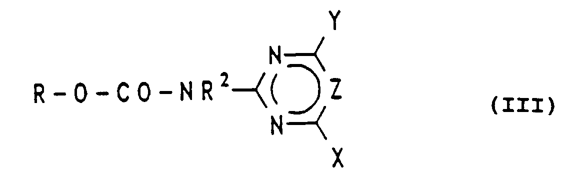 in which R is unsubstituted or substituted phenyl, or
b) reacting a sulfochloride of the formula (IV) 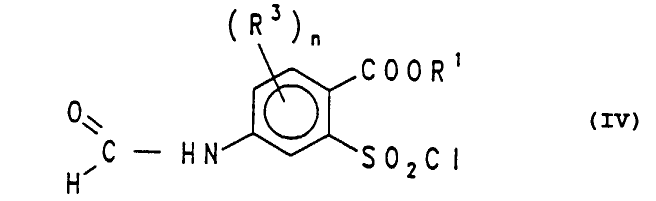 with a heterocyclic amine of the formula (V) 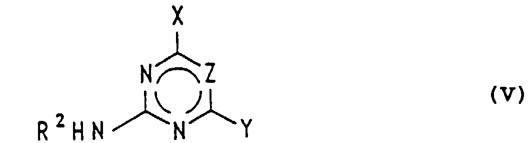 in the presence of a cyanate, or
c) formylating a sulfonylurea of the formula (VI) 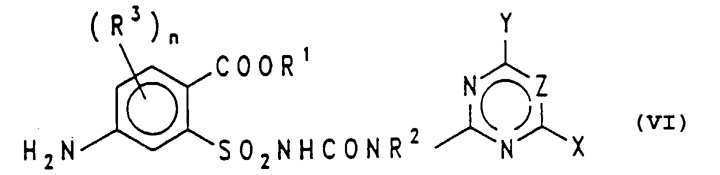 or
d) reacting a sulfonamide of the formula (II) with a (thio)isocyanate of the formula (VII) in the presence of a suitable base 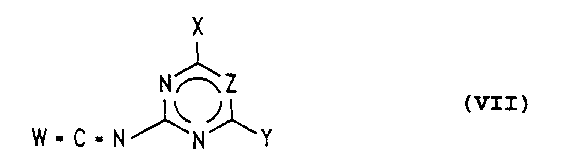
where in the above formulae (II) to (VII) the radicals R¹, R², R³, W, X, Y and Z and also the index n are as defined in formula (I) and in the process variants a), b) and c) compounds are first obtained in which W is an oxygen atom.

5. A herbicidal or plant growth-regulating composition, which comprises at least one compound of the formula (I) as claimed in one of claims 1 to 3 and formulation auxiliaries conventionally used in crop protection.

6. A method of controlling harmful plants or of regulating the growth of plants, which comprises applying an effective amount of at least one compound of the formula (I) as claimed in one of claims 1 to 3 to the harmful plants or plants, the seeds of these plants or the area on which the plants grow.

7. The use of the compounds of the formula (I) as claimed in one of claims 1 to 3 as herbicides or plant growth regulators.

8. A compound of the formula (II) as defined in claim 4.

## Revendications

1. Composés de formule (I), 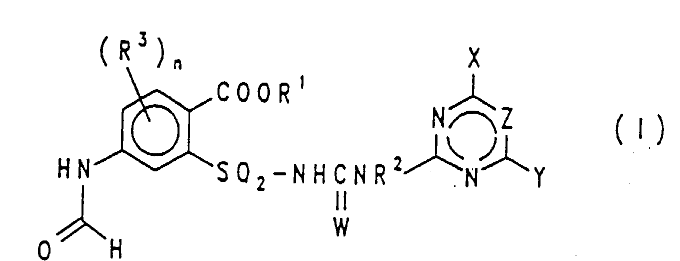 dans laquelle
R¹ représente un atome d'hydrogène, un groupe hydrocarboné substitué ou non substitué ou un groupe hétérocyclique substitué ou non substitué,
R² représente un atome d'hydrogène, un groupe alkyle en C₁-C₆, ou alkoxy en C₁-C₆,
R³ représente un atome d'halogène, un groupe alkyle en C₁-C₆, alkoxy en C₁-C₆, halogénoalkyle en C₁-C₆, halogénoalkoxy en C₁-C₆, NO₂, CN, NH₂, mono ou dialkylamino en C₁-C₄, et est même respectivement indépendant des autres radicaux R³, lorsque n vaut 2 ou 3,
n est 0, 1, 2 ou 3,
W représente un atome d'oxygène ou un atome de soufre,
X, Y représentent indépendamment l'un de l'autre un atome d'halogène, un groupe alkyle en C₁-C₆, alkoxy en C₁-C₆, alkylthio en C₁-C₆, chacun des trois derniers radicaux cités étant non substitué ou substitué par un ou plusieurs résidus choisis parmi un atome d'halogène, un groupe alkoxy en C₁-C₄ et alkylthio en C₁-C₄, ou un groupe cycloalkyle en C₃-C₆, alcényle en C₂-C₆, alcynyle en C₂-C₆, alcényloxy en C₂-C₆, alcynyloxy en C₂-C₆, mono- ou di-(alkyle en C₁-C₄)-amino et
Z représente un groupe CH ou un atome d'azote.

2. Composés de formule (I) selon la revendication 1, caractérisés en ce que,
R¹ représente un atome d'hydrogène, un groupe alkyle en C₁-C₆, alcényle en C₂-C₆, alcynyle en C₂-C₆, cycloalkyle en C₃-C₆, dans lequel les quatre derniers radicaux cités sont non substitués ou substitués par un ou plusieurs groupes choisis parmi un atome d'halogène, CN, un groupe alkoxy en C₁-C₃, halogénoalkoxy en C₁-C₃, cycloalkyle en C₃-C₆, alkylthio en C₁-C₃, alkylsulfinyle en C₁-C₃, alkylsulfonyle en C₁-C₃, phényle non substitué, phényle substitué, un radical hétérocyclique non substitué et un radical hétérocyclique substitué, dans le cas de groupes cycliques également par un groupe alkyle en C₁-C₄, ou un groupe hétérocyclique avec 3, 4, 5, 6 ou 7 atomes dans le cycle et un ou plusieurs atomes choisis parmi O, N et S comme atome d'hétérocycle, le groupe étant non substitué ou substitué par un ou plusieurs résidus choisis parmi un atome d'halogène, un groupe alkyle en C₁-C₃ et halogénoalkyle en C₁-C₃,
R² représente un atome d'hydrogène, un groupe alkoxy en C₁-C₃ ou alkyle en C₁-C₃,
R³ représente un atome d'halogène, un groupe alkyle en C₁-C₃, alkoxy en C₁-C₃, halogénoalkyle en C₁-C₃, halogénoalkoxy en C₁-C₃, NO₂, CN, NH₂, NHCH₃, N(CH₃)₂,
n est 0, 1, 2 ou 3,
W représente un atome d'oxygène ou un atome de soufre,
X, Y représentent indépendamment l'un de l'autre un atome d'halogène, un groupe alkyle en C₁-C₄, alkoxy en C₁-C₄, alkylthio en C₁-C₄, chacun des trois derniers radicaux cités étant non substitué ou substitué par un ou plusieurs résidus choisis parmi un atome d'halogène, un groupe alkoxy en C₁-C₄ et alkylthio en C₁-C₄, ou cycloalkyle en C₃-C₆, alcényle en C₂-C₄, alcynyle en C₂-C₄, alcényloxy en C₂-C₄, alcynyloxy en C₂-C₄, mono- ou di-(alkyle en C₁-C₂)-amino et
Z représente un groupe CH ou un atome d'azote.

3. Composés de formule (I) selon la revendication 1 ou 2, caractérisés en ce que,
R¹ représente un groupe alkyle en C₁-C₆, alcényle en C₂-C₆, alcynyle en C₂-C₆, cycloalkyle en C₃-C₆, cycloalkylalkyle en C₄-C₈, phényl-alkyle en C₁-C₆, chacun des six derniers résidus cités étant non substitué ou substitué par un ou plusieurs résidus choisi parmi un atome d'halogène, CN, OCH₃, OC₂H₅, OCF₃, SO₂CH₃ et dans le cas de groupes cycliques également par un groupe alkyle en C₁-C₃,
ou un résidu de formules A₁ à A₇, 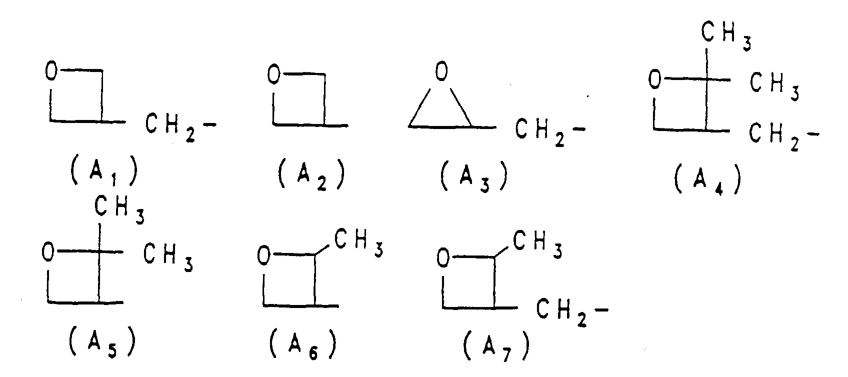
R² représente un atome d'hydrogène ou un groupe CH₃,
R³ représente un atome d'halogène, un groupe CH₃, C₂H₅, OCH₃, OC₂H₅, CF₃, CCl₃, OCF₃, OCHF₂ ou N(CH₃)₂,
n est 0, 1 ou 2.

4. Procédé de préparation de composés de formule (I) selon la revendication 1, caractérisé en ce que,
a) on met à réagir un composé de formule (II) 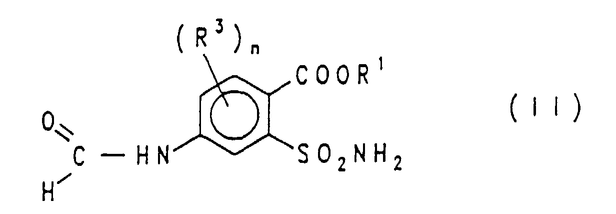 avec un carbamate hétérocyclique de formule (III), 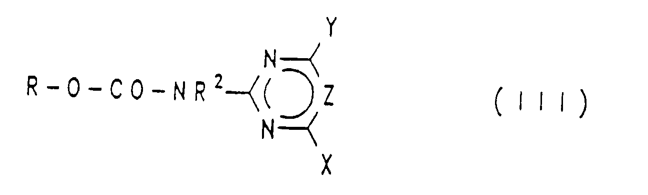 dans lequel R représente un groupe phényle substitué ou non substitué, ou
b) on met à réagir un sulfochlorure de formule (IV) 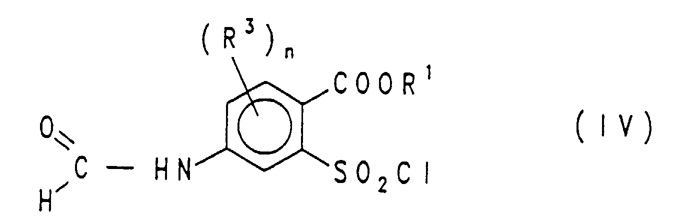 avec une amine hétérocyclique de formule (V) 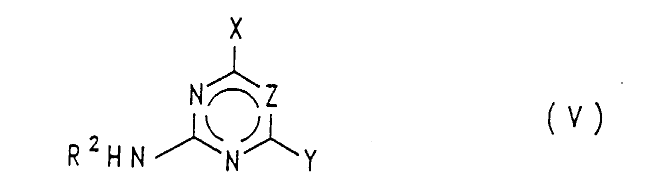 en présence d'un cyanate, ou
c) on effectue la formylation d'une sulfonylurée de formule (VI) 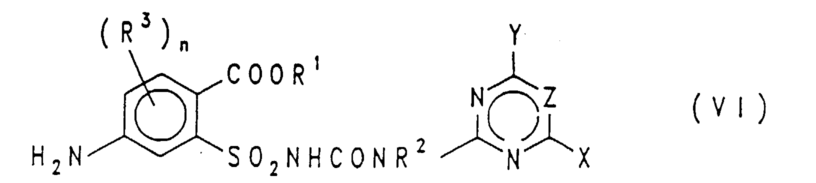 ou
d) on met à réagir un sulfonamide de formule (II) avec un (thio)isocyanate de formule (VII) en présence d'une base appropriée 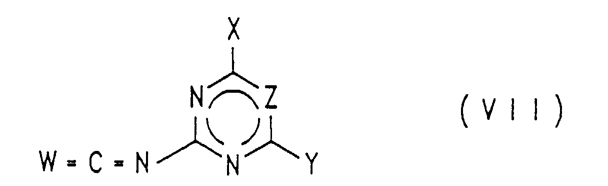
formules (II) à (VII) ci-dessus , dans les groupes R¹, R², R³, W, X, Y et Z ainsi que l'indice n sont définis comme dans la formule (I) et dans les variantes de procédé a), b), c), on obtient d'abord des composés dans lesquels W représente un atome d'oxygène.

5. Agent herbicide ou régulateur de croissance des végétaux, caractérisé en ce qu'il contient au moins un composé de formule (I) selon l'une des revendications 1 à 3 et des additifs de formulation usuels dans la protection des plantes.

6. Procédé pour combattre les plantes nuisibles ou pour réguler la croissance de végétaux, caractérisé en ce que, l'on applique une quantité efficace d'au moins un composé de formule (I) selon l'une des revendications 1 à 3 sur les plantes nuisibles et les plantes, leurs semences ou la surface sur laquelle poussent les végétaux.

7. Utilisation des composés de formule (I) selon l'une des revendications 1 à 3 comme herbicides ou régulateurs de croissance de végétaux.

8. Composés de formule (II), tels qu'ils sont définis dans la revendication 4.
